# EUROPEAN PATENT SPECIFICATION

(11) **EP 1 276 518 B1**
(45) Date of publication and mention of the grant of the patent: **13.09.2006**
(21) Application number: 00987618.6
(22) Date of filing: 18.12.2000
(51) Int. Cl.: A61M 1/00

(54) **PULSATILE PUMP OR FLOW MODULATOR FOR EXTRACORPOREAL CIRCULATION**
PULSIERENDE PUMPE ODER STRÖMUNGSMODULATOR FÜR EXTRAKORPORALEN KREISLAUF
POMPE OU MODULATEUR PULSATOIRE EXTRACORPOREL A AJOUTER A UN SYSTEME DE PERFUSION A ECOULEMENT EXTRACORPOREL CONTINU

(30) Priority: 16.12.1999 IT CS990022
(43) Date of publication of application: 22.01.2003
(73) Proprietor: Tecnobiomedica S.p.A., 00040 Pomezia (RM) (IT)
(72) Inventor: Arabia, Maurizio, 00137 Roma (IT); Danieli, Guido A., 87036 Arcavacata di Rende (IT)
(74) Representative: Bosotti, Luciano
(86) International application number: PCT/IT2000/000526
(87) International publication number: WO 2001/043797

(56) References cited:
- US-A- 4 610 656
- US-A- 4 976 593
- US-A- 5 300 015
- US-A- 5 916 191

## Description

### General Description of the field of application of the invention

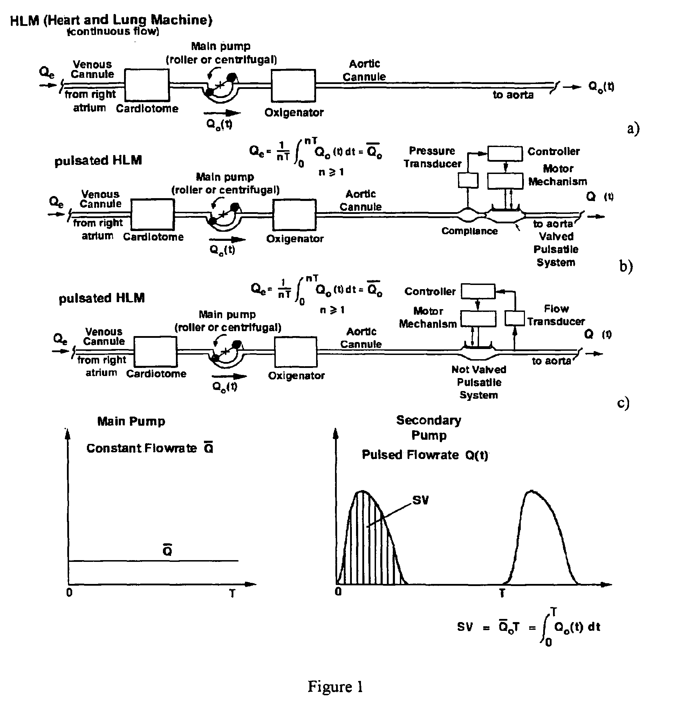

In some cardiosurgery operations it is necessary the use of a Heart Lung Machine in which continuous flow pumps are used (Fig. 1 a). But the human body is used to the pulsatile blood flow, produced by the heart. Results obtained in total artificial heart and in ventricular assist devices applications, which generate pulsatile flow and allows survival more than one year, suggest that the pulsatile flow is definitely not dangerous and better than continuous flow. Moreover the needs to use cardiopulmonary bypass during the always longer and complex surgery operations, suggest the opportunity to switch from continuous to pulsatile flow.

A way is to replace the continuous flow pump used in the HLM with a pulsatile pump. Another way is to add a pulsatile pump in the terminal part of an HLM, matching the pulsatile pump behaviour to the HLM behaviour, to obtain that the average (on one or more periods) pulsatile pump flow could be equal to the flow of the continuous flow pump and that the pressures in the circuit between the pumps could be correct. A third way is to use, always in the terminal part of an HLM, a flow modulator device which, properly filling and ejecting, produces a negative or positive flowrate that is subtracted or added to the continuous flow, so realizing the desired correct flow waveform and, as a consequence, the correct pressure waveform in aorta. If the continuous flow pump has a pressure depending flow (for instance the centrifugal pump Medtronic Bio-Medicus), the flow modulator must be properly controlled in order to compensate the flow fluctuations of the continuous flow pump. These are due to the pulsatility of the aortic pressure which, coming back through the aortic cannula, produces a pulsatile load to the output of the continuous flow pump.

In all the solutions the flowrate of the pulsatile pump or of the modulator, which is given by HR*SV (HR=pump or modulator frequency; SV= pump or modulator stroke volume), can be regulated by modifying HR and/or SV.

In the first solution, (one pump system), the pulsatile flow is present in the oxigenator and in the cannula connecting the oxigenator to aorta. Therefore the cannula's inertance influence (which is greater than its compliance influence) and the oxigenator mechanical impedance make necessary high positive and negative pressure peaks in the pump, during the corresponding flow increasing and decreasing phases, to obtain the correct pulsatile flow waveform. But while it is possible to reproduce the increasing flow phase by proper positive pressure peak in the pump, it can result impossible to reproduce the decreasing flow phase if the negative pressure peak, which is necessary to reduce the blood velocity in the aortic cannula, is lower than the blood vapour tension. The consequence is the difficulty to generate the correct flow waveform (necessary to produce the correct aortic pressure waveform) and the possibility to damage the blood. To compensate this problem should be necessary to use a cannula (from oxigenator to aorta) having an increased distributed compliance, and to control the pump, according to the desired and the measured (immediatly before the input to aorta) flow waveforms and to the average flow value selected from the perfusionist.

This principle could be applied also to the second and the third solution, allowing to put the pulsatile pump or the modulator immediatly after the oxigenator: in these cases the control system of the pulsatile pump or of the modulator has to compensate only the cannula. These solutions, however, seem to be complex and expensive, particularly the first one which requires to compensate not only the cannula, but also the oxygenator.

The document US-A-4 610 656 discloses a fully portable semi-automatic mechanical heart-lung substitution system comprising inter alia an oxygenator, a compliant reservoir and a pulsatile left heart pump, whose output is connected through tubing to an arterial cannula which may be inserted into the patient's arterial system.

The pressure within said compliant reservoir may automatical control stroke volume of said pump (58), and in the automatic mode, the output of the left heart pump (58) returns the same volume of blood to the patient and at the same rate as is pumped from the patient's vena cava right heart pump.

The invention is defined in Claim 1. The subclaims set out particular embodiments of the invention.

Aim of the invention is the development of an active or passive device, which can be added at the end of any continuous flow HLM, immediately before the connection to aorta (or immediatly after the oxygenator, but using a cannula having a radial elasticity, and as a consequence a proper distributed compliance, plus a proper control system, so that the flow at the output of this device, going to aorta, is pulsatile according to a physiological or, anyway, adequate waveform.

These systems can be active or passive type.

The active systems use any rotational or linear motion electric motors, by which it is possible to produce a reciprocating movement of a piston.

The passive systems use the energy supplied by the continuous flow pump of the HLM. In this case the basic elements of the device are a compliance and an electrovalve. When the electrovalve is closed, blood coming from the continuous flow pump is stored in the compliance, where - as a consequence - the pressure increases. When the electrovalve opens, in the output there is a flow to aorta that is the sum of the continuous flow coming from the HLM and of the flow produced by the discharge of the compliance. If a proportional electrovalve is used, it is possible to control the instantaneous flow to aorta according to the desired waveform.

These devices can operate and be controlled in different ways, according to the desired performance

If the device is a pulsatile pump (active, pulsatile system having input and output unidirectional valves) it can operate as an artificial ventricle; moreover an artificial atrium is needed at its input in order to adapt the continuous (or about continuous) flow coming from the oxigenator to the pulsatile filling flow of the pulsatile pump. The stability of the system (average flowrates balance between the continuous or about continuous flow and the pulsatile flow) and proper values for the artificial atrium pressure, can be obtained by using the artificial atrium pressure as a control variable of the pulsatile pump average flow (particularly of HR when the pump is working with a fixed SV), operating similarly to the natural heart (Starling law). According to this, the pump flowrate is a function of the atrial pressure and increases (decreases) when atrial pressure increases (decreases). So, when the value of the continuous flow is modified by the perfusionist (for instance increases), in the atrium starts an increase of pressure, till a value -which for- the increasing pulsatile pump average flowrate is equal to the continuous or to the average of the about continuous flow. This device allows different operation modes: tipical is the "full fill - full empty" mode. When the ventricle is completely filled, starts its ejection with a duration which is depending on its previous filling time and on the systole/ diastole ratio. The sum of the ejection time and of the filling time is the period, from which is derived HR. In this approach the possible available knowledge of the flowrate, selected by the perfusionist, is not necessary to the control system of the pulsatile pump (Fig. 1 b).

If the device is a flow modulator (active, pulsatile system without input and output unidirectional valves), it fills with a flowrate equal to that one coming from the continuous flow pump, so annulling the flow to aorta; then it ejects the filling volume with a proper flow waveform that, combined with the flow coming from the continuous flow pump, produces the physiological or, anyway, adequate flow waveform to aorta.

If the modulator is working with a fixed stroke volume SV and flowrate waveform, its frequency, (that, for instance, can be varied between 60 and 120 beats/min) depends on the average flowrate of the continuous flow pump. In this solution it is necessary to measure the instantaneous flowrate at the input or output section of the modulator (better if in the section of the cannula, immediatly before the input to aorta) and use this information to control the motion of the piston and the frequency HR (Fig. 1 c). If the continuous flow pump is a roller pump (approximately its flow rate is not depending on its pressure load) and if limited performances are accepted, the control system can be semplified using an approximate knowledge of the roller pump flowrate to control the HR of the modulator. In this case, if the used HR is a little lower (higher) than the correct value, there is a little flow from modulator to aorta (from aorta to modulator) during the time interval in which the the flow should be zero.

This system is more simple than the pulsatile pump: there are no valves, no artificial atrium; moreover the necessary ejected volume (stroke volume) is lower than the one in the pulsatile pump.

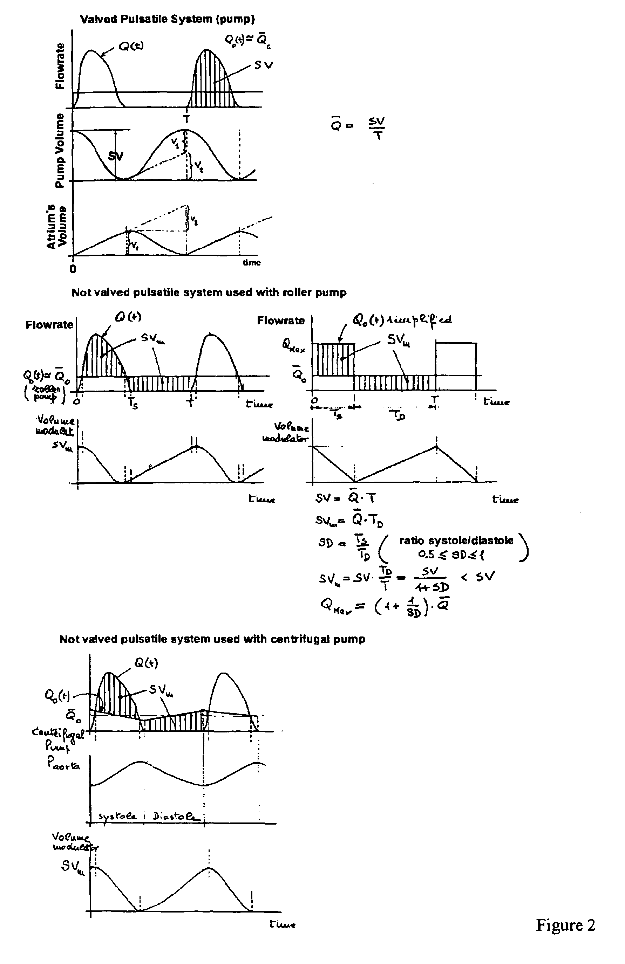

Fig.2 shows the basic fluid-dynamic properties of the two systems.

An interesting characteristic of these devices is that it is possible to stop them while the HLM is working, so restoring the continuous flow, if desired.

The proposed devices can operate correctly with any extracorporeal system using any type of continuous (constant or about constant) flow pump. Another relevant characteristics are the reduced dimensions of the device, as this must be positioned hear the patient for a better operation mode, and a reduced priming volume.

### Description of the preferred embodyment

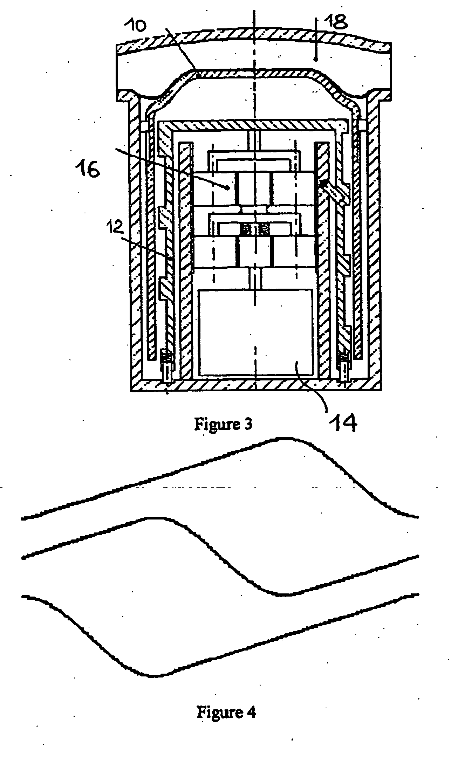

The preferred, but not the only, layout is shown in Fig. 3. The piston 10, which can't rotate, is is moved in a reciprocating fixed linear stroke mode by three cams 12 having a proper profile, producing the ejection motion and the filling motion. The actuator is an electric motor 14 whose angular velocity is properly reduced by a gear set 16. A constant angular velocity of the motor, and then of the three cams on which are rolling three standard bearings, produces a fixed flow profile. If desired it is possible to modify the flow profile by using a proper angular velocity profile, so to obtain the physiological or - more generally - the adequate to the patient, flow profile for the different working modes. The piston 10 squeezes and releases a deformable blood chamber (ventricular sac 18), made by biocompatible material, connected to the oxygenator and to the patient. In the chamber, blood is continuously coming from the oxygenator, pushed by the continuous or about continuous flow pump located before the oxygenator; and from the chamber is coming out blood -with a pulsatile flow- which is going to the patient.

The frequency HR of the modulator, having a properly selected constant stroke volume SVm, is correlated to the average flowrate *Q̅* of the HLM pump, selected by the perfusionist, with a relation as HR = k**Q̅*. If, for instance, 4<=*Q̅* <=8 liters/min and k=15 (beats/min)/(liters/min), the frequency is ranging from 60 to 120 beats/min. Moreover if the systole/diastole ratio of the pulsatility is assumed equal to 0.5 and constant for semplicity, it follows that SVm= about 45 ml.

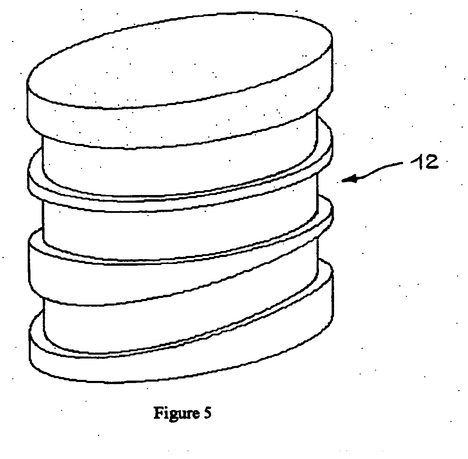

Fig. 4 shows the profiles of three possible cams 12, having a relative mechanical angular phase relation equal to 2 π / 3. In Fig. 5 it is shown the inner of the mechanism of Fig.3, which produces the motion law indicated in Fig. 4. It is possible to combine the three cams 12 in one (reducing the angular velocity to 1/3 of the previous value), so obtaining a more compact device.

For simplicity in the following we name ECP the continuous flow pump (peristaltic pump, roller, centrifugal or similar) of the extracorporeal systems (HLM) used in cardiosurgery; and we name PulsaDev (i.e, pulsatile device) our device (active systems as pulsatile pump or modulator, or passive systems).

## Claims

1. A pulsatile device located between the output of the oxygenator and the input to the patient in a heart lung machine, to convert the continuous - or nearly continuous - flow, coming from the oxygenator into a pulsatile physiological flow, going to the patient, using a flow modulator comprising a variable volume chamber (18) without valves, said modulator (18) having a flow transducer located at its input or output section and a control system which matches the operation mode of the device as selected by the perfusionist, to the flow of the extracorporeal circuit pump, and to the need of correct flow waveform in the aorta.

2. The device of Claim 1, has its average flow selected directly from the perfusionist or automatically from the extracorporeal circuit pump.

3. The device of any of Claims 1-2, including a piston (10) to squeeze and release said variable volume chamber (18), wherein the piston motion is generated mechanically by the rotation of a proper cam (12), which produces the desired motion law using a motor (14), working with constant angular velocity, whose value depends on the required average flowrate in the cycle.

4. The device of any of Claims 1-2, including a piston (10) to squeeze and release said variable volume chamber (18), wherein the piston motion is generated by a velocity controlled motor (14), connected to the piston (10) by a mechanism (16), whose characteristics are compensated to obtain the desired motion law.

5. The device of any of Claims 1-2, including a piston (10) to squeeze and release said variable volume chamber (18), wherein the piston motion is generated by a linear motion motor (10) controlled in position or in position and velocity, said motor being connected directly to the piston (10) to obtain the desired motion law.

## Patentansprüche

1. Pulsierende Vorrichtung, die zwischen dem Ausgang des Oxygenators und dem Eingang in den Patienten in einer Herz-Lungen-Maschine angeordnet ist, um die kontinuierliche oder nahezu kontinuierliche Strömung, die von dem Oxygenator kommt, unter Verwendung eines Strömungs-Modulators, der eine Kammer (18) mit veränderlichem Volumen ohne Ventile umfasst, in eine pulsierende physiologische Strömung, die zu dem Patienten gelangt, umzuwandeln, wobei der Modulator (18) einen Strömungssensor, der an seinem Eingangs- oder Ausgangsabschnitt angeordnet ist, sowie ein Steuerungssystem hat, das den durch den Perfusionisten ausgewählten Funktionsmodus der Vorrichtung an die Strömung der Extrakorporalkreislauf-Pumpe und an die Notwendigkeit richtiger Strömungs-Wellenform in der Aorta anpasst.

2. Vorrichtung nach Anspruch 1, bei der die durchschnittliche Strömung direkt von dem Perfusionisten oder automatisch von der Extrakorporalkreislauf-Pumpe ausgewählt wird.

3. Vorrichtung nach einem der Ansprüche 1-2, die einen Kolben (10) zum Zusammendrücken und Freigeben der Kammer (18) mit variablem Volumen enthält, wobei die Kolbenbewegung mechanisch durch die Drehung eines entsprechenden Nockens (12) erzeugt wird, der den gewünschten Bewegungsablauf unter Verwendung eines Motors (14) herstellt, der mit konstanter Winkelgeschwindigkeit arbeitet, deren Wert von der erforderlichen durchschnittlichen Strömungsgeschwindigkeit in dem Zyklus abhängt.

4. Vorrichtung nach einem der Ansprüche 1-2, die einen Kolben (10) zum Zusammendrücken und Freigeben der Kammer (18) mit variablem Volumen enthält, wobei die Kolbenbewegung durch einen geschwindigkeitsgesteuerten Motor (14) erzeugt wird, der mit dem Kolben (10) durch einen Mechanismus (16) verbunden ist, dessen Eigenschaften kompensiert werden, um den gewünschten Bewegungsablauf zu erzielen.

5. Vorrichtung nach einem der Ansprüche 1-2, die einen Kolben (10) zum Zusammendrücken und Freigeben der Kammer (18) mit variablem Volumen enthält, wobei die Kolbenbewegung durch einen Linearbewegungsmotor (10) erzeugt wird, dessen Position oder Position und Geschwindigkeit gesteuert werden, wobei der Motor direkt mit dem Kolben (10) verbunden ist, um den gewünschten Bewegungsablauf zu erzielen.

## Revendications

1. Dispositif pulsatile situé entre la sortie de l'oxygénateur et l'entrée vers le patient dans un coeur-poumon artificiel, pour convertir le flux continu ou quasi-continu provenant de l'oxygénateur en un flux physiologique pulsatile dirigé vers le patient utilisant un modulateur de flux comprenant une chambre (18) à volume variable sans valves, ledit modulateur (18) ayant un transducteur de flux situé au niveau de sa section d'entrée ou de sortie et un système de commande qui accorde le mode de fonctionnement du dispositif sélectionné par le perfusionniste au flux de la pompe de circuit extracorporel et au besoin d'une forme d'onde de flux correcte dans l'aorte.

2. Dispositif selon la revendication 1 dont le flux moyen est sélectionné directement par le perfusionniste ou automatiquement depuis la pompe du circuit extracoporel.

3. Dispositif selon l'une quelconque des revendications 1 - 2, incluant un piston (10) pour écraser et étendre ladite chambre (18) à volume variable, le mouvement du piston étant généré mécaniquement par rotation d'une came adaptée (12), qui produit la loi de mouvement désirée en utilisant un moteur (14), fonctionnant à une vitesse angulaire constante, dont la valeur dépend du débit moyen requis dans le cycle.

4. Dispositif selon l'une quelconque des revendications 1 - 2, incluant un piston (10) pour écraser et étendre ladite chambre (18) à volume variable, le mouvement du piston étant généré par un moteur (14) de vitesse contrôlée, relié au piston (10) par un mécanisme (16) dont les caractéristiques sont compensées pour obtenir la loi de mouvement désirée.

5. Dispositif selon l'une quelconque des revendications 1-2, incluant un piston (10) pour écraser et étendre ladite chambre (18) à volume variable, le mouvement du piston étant généré par un moteur (10) à mouvement linéaire contrôlé en position ou en position et en vitesse, ledit moteur étant relié directement au piston (10) pour obtenir ladite loi de mouvement désirée.
